# FASCICULE DE BREVET EUROPEEN

(11) **EP 3 630 071 B1**
(45) Date de publication et mention de la délivrance du brevet: **22.07.2026**
(21) Numéro de dépôt: 18735643.1
(22) Date de dépôt: 22.05.2018
(51) Int. Cl.: A61K 9/20

(54) **COMPRIMÉ SÉCABLE**
GEKERBTE TABLETTE
SCORED TABLET

(30) Priorité: 23.05.2017 FR 1754581
(43) Date de publication de la demande: 08.04.2020
(73) Titulaire: Ceva Santé Animale, 33500 Libourne (FR)
(72) Inventeur: MALLO, Nadège, 53000 Laval (FR); JOLIVET, Philippe, 53000 Laval (FR); VENEL, Mickael, 53000 Laval (FR); GARCIA, Rosita, 53000 Laval (FR)
(74) Mandataire: Plasseraud IP
(86) Numéro de dépôt international: PCT/FR2018/051223
(87) Numéro de publication internationale: WO 2018/215723

(56) Documents cités:
- EP-A1- 1 568 362
- US-B1- 7 985 419

## Description

L'invention a trait à un comprimé sécable pouvant être facilement coupé, en deux ou quatre parties, par simple pression exercée sur ledit comprimé. L'invention a plus particulièrement pour objet un tel comprimé sécable à usage vétérinaire, notamment pour chiens et chats.

Les comprimés sont utilisés depuis longtemps pour administrer par voie orale une composition pharmaceutique solide à un sujet. Les comprimés sont généralement avalés ou croqués, afin de libérer la ou les substances actives qu'ils contiennent dans l'organisme. Le plus souvent, les comprimés sont obtenus en agglomérant par compression un volume de particules contenant la ou les substances actives. Aussi, un type de comprimé donné, de forme et de poids définis, comprend systématiquement la même quantité de substances actives.

Dans la mesure où la quantité de substances actives à administrer peut varier d'un sujet à un autre, notamment en fonction du poids du sujet, les laboratoires ont développé des comprimés sécables, permettant de diviser en moitié ou en quart, ledit comprimé et par là la quantité de substances actives.

Dans le domaine vétérinaire, de par la diversité en poids des animaux, la sécabilité des comprimés revêt une importance particulière. En effet, un même comprimé peut être, par exemple, à destination des chiens et des chats, qui sont de corpulences très variables. Il est donc nécessaire de pouvoir adapter la dose de substances actives à administrer.

Les comprimés sécables présentent le plus souvent une ou deux lignes creusées dans l'épaisseur desdits comprimés pour faciliter la coupe le long desdites lignes. Cependant, l'étape de coupe est souvent malaisée. La pression à exercer est d'autant plus difficile à appliquer que le comprimé est de petite taille et qu'il n'est pas simple de le tenir entre les doigts. Il en résulte une coupe imprécise et irrégulière, entraînant de fait une imprécision dans les quantités de substances actives contenues dans les portions de comprimé.

On connaît du document US7985419 B1, un comprimé pharmaceutique sécable, non susceptible de rupture prématurée et qui peut être relativement facilement divisé en sous-portions, les sous-portions contenant des quantités précises et constantes de produits pharmaceutiques. Pour cela, ce comprimé comporte une ou plusieurs encoches de sécabilité pour former des fragments individuels, les encoches de sécabilité étant toutes discontinues à leur intersection pour permettre à un consommateur de diviser le comprimé en deux ou plusieurs sous-portions.

On connaît également du document EP 1 568 362 A1 un comprimé qui peut être divisé en quatre. Il se compose d'un élément de comprimé de forme quadratique, d'une face supérieure et d'une face inférieure. La face inférieure est convexe et la face supérieure est plane et présente des lignes de sécabilité en forme de V qui sont perpendiculaires l'une à l'autre.

L'invention a pour objectif de résoudre au moins partiellement les problèmes associés à la sécabilité des comprimés, en fournissant un comprimé apte à être divisé en deux ou en quatre parties identiques en exerçant une ou deux pressions sur ledit comprimé, ce comprimé étant configuré pour autoriser une première casse de ce dernier selon une direction donnée du comprimé.

Pour cela, le comprimé selon l'invention présente une face globalement concave, destinée à être appliquée contre une surface plane, et une face convexe sur laquelle la pression doit être exercée. Deux lignes de coupe perpendiculaires l'une à l'autre sont creusées dans la face concave et se prolongent jusque dans le pourtour extérieur du comprimé, délimitant ainsi les demis et quarts de comprimé. La forme du comprimé associée aux lignes de coupe qui s'étendent de part et d'autre du comprimé participent à assurer une coupe précise et facile. Le comprimé selon l'invention permet d'obtenir des demis ou quarts de comprimés sans ou avec peu de dispersion de poids. Avantageusement, le comprimé selon l'invention présente en outre une forme ronde, ou circulaire cylindrique, rendant la production à l'échelle industrielle plus aisée. La fabrication par compression de tels comprimés de forme ronde permet un gain en termes de productivité de 30 à 50% par rapport à d'autres formes de comprimés plus complexes, telle que la forme en trèfle.

L'invention a donc pour objet un comprimé sécable tel que défini dans la revendication 1.

Selon l'invention, on désigne par « face inférieure », la face destinée à être appliquée contre une surface lors de l'étape de coupe du comprimé, par opposition à la « face supérieure ».

Dans un mode de réalisation particulier, le rayon de courbure de la partie concave de la face inférieure est compris entre 150° et 170°, préférentiellement égal à 165°, +/- 5°.

Les gorges de sécabilité et les fentes de sécabilité sont creusées dans l'épaisseur du comprimé, de manière à délimiter quatre triangles de forme et de taille identiques ou sensiblement identiques. L'« épaisseur » du comprimé s'entend de la dimension s'étendant perpendiculairement au plan de la face inférieure ou supérieure du comprimé.

Selon l'invention, le comprimé peut être divisé en deux demi-comprimés en exerçant une première pression sur la face convexe. Le comprimé est cassé en deux le long d'une première gorge de sécabilité. Si nécessaire, chaque demi-comprimé peut être à nouveau cassé en deux, le long de la seconde gorge de sécabilité, en exerçant une pression sur la face convexe dudit demi-comprimé. On obtient ainsi quatre parties de comprimé de forme sensiblement triangulaire, parfaitement identiques.

Avantageusement, le comprimé selon l'invention a une forme circulaire cylindrique. Une telle conformation facilite notamment l'emballage industriel des comprimés sous blister. Dans un autre mode de réalisation, le comprimé peut avoir une forme ovale, carrée, rectangulaire, etc.

Dans un mode de réalisation particulier, les gorges de sécabilité de la face inférieure ont une section en V. Avantageusement, la section en V présente un angle d'ouverture compris entre 40° et 100°, préférentiellement égal à 80° +/- 5°. La section en V s'entend selon un plan de coupe perpendiculaire au plan de la face inférieure du comprimé.

Avantageusement, les gorges de sécabilité de la face inférieure ont une profondeur comprise entre 1/5 et 3/10 de l'épaisseur du comprimé. Par « profondeur », on entend la dimension s'étendant dans un plan perpendiculaire au plan de la face inférieure

Dans un mode de réalisation particulier, l'une des gorges de sécabilité est continue et l'autre gorge de sécabilité est discontinue. Une telle discontinuité favorise la première casse du comprimé le long de la gorge de sécabilité continue.

Préférentiellement, la gorge de sécabilité discontinue s'interrompt en amont de l'intersection des gorges de sécabilité. La gorge de sécabilité discontinue peut autrement présenter une ou plusieurs interruptions successives, jusqu'à former par exemple une ligne en pointillés.

Dans un mode de réalisation particulier, les deux gorges de sécabilité de la face inférieure ont des longueurs différentes. Par « longueur », on étend la dimension s'étendant selon la plus grande dimension du comprimé. Cette conformation favorise la première casse du comprimé selon la gorge de sécabilité la plus courte. Par exemple, deux des fentes de sécabilité s'étendant dans le prolongement de l'une des gorges de sécabilité sont creusées plus profondément dans le diamètre du comprimé que les deux autres fentes de sécabilité, de manière à réduire d'autant la longueur de la gorge de sécabilité associée.

Selon l'invention, les gorges de sécabilité divisent le comprimé en quatre triangles sensiblement identiques. Dans un mode de réalisation particulier, la face inférieure de chacun des triangles présente une arête s'étendant depuis le bord extérieur du comprimé, vers une gorge de sécabilité, à l'intersection ou en amont de l'intersection desdites gorges de sécabilité. Par « arête », on entend angle saillant formé par la rencontre des deux surfaces bordant ledit angle. De manière alternative, la face inférieure de chacun des triangles est bombée. Ainsi, la face inférieure du comprimé présente des surfaces d'appui discontinues lorsqu'elle est en contact avec une surface plane. La face inférieure présente alors une concavité partielle, s'étendant principalement le long d'une des gorges de sécabilité. De manière alternative, la face inférieure présente une surface concave uniforme.

Dans un mode de réalisation particulier, un bord extérieur de la face inférieure est biseauté, le long de la paroi latérale.

Dans un mode de réalisation particulier, les fentes de sécabilité de la paroi latérale ont une section en V. Avantageusement, la section en V présente un angle d'ouverture compris entre 70° et 120°, préférentiellement égal à 90° +/- 5°. La section en V s'entend selon un plan de coupe parallèle au plan de la face inférieure du comprimé. Dans un exemple de réalisation, les angles d'ouverture des fentes de sécabilité sont tous égaux. Dans un autre exemple de réalisation, les angles d'ouverture des fentes de sécabilité sont égaux deux par deux. Plus précisément, les angles d'ouverture des fentes de sécabilité s'étendant dans le prolongement d'une même gorge de sécabilité sont égaux, mais différents des angles d'ouverture des fentes de sécabilité s'étendant dans le prolongement de l'autre même gorge de sécabilité.

Le comprimé sécable selon l'invention présente avantageusement une plus grande dimension comprise entre 10 et 22 mm, préférentiellement entre 16 et 20 mm, plus préférentiellement de 18 mm. Dans un mode de réalisation particulier, le comprimé selon l'invention présente une plus grande dimension de 10 mm, 12 mm, 14 mm, 16 mm, 18 mm, 20 mm, ou 22 mm, +/- 0,5 mm. Dans le cas où le comprimé est de forme générale circulaire cylindrique, la plus grande dimension s'entend du plus grand diamètre dudit comprimé. L'épaisseur d'un tel comprimé est comprise entre 0,4 et 1 mm, préférentiellement 0,45 et 0,85 mm, et la profondeur des gorges de sécabilité est comprise entre 0,1 et 0,3 mm.

L'invention sera mieux comprise à la lecture de la description qui suit et à l'examen des figures qui l'accompagnent. Celles-ci sont présentées à titre informatif et nullement limitatif de l'invention. Les figures représentent :
Figure 1 : Une représentation schématique vue de dessous (A) et vue de dessus (B) d'un comprimé sécable selon un exemple de réalisation de l'invention ;
Figure 2 : Deux représentations schématiques (A et B) vue de côté d'un comprimé sécable selon un exemple de réalisation de l'invention.

Un but de l'invention est de fournir un comprimé sécable pouvant être coupé si besoin en deux ou en quatre parties égales, afin de pouvoir aisément adapter la quantité de substance active à administrer en fonction de la posologie. Un même type de comprimé peut ainsi être administré à différents utilisateurs, et plus particulièrement différents animaux, présentant notamment une large gamme de poids, puisqu'il est possible d'administrer la totalité, la moitié ou le quart de la dose de substances actives initialement contenue dans le comprimé. Le comprimé sécable selon l'invention présente des gorges et fentes de sécabilité permettant de garantir une coupe homogène, par simple pression, pour obtenir des demis ou quarts de comprimé comprenant une quantité contrôlée de substance active.

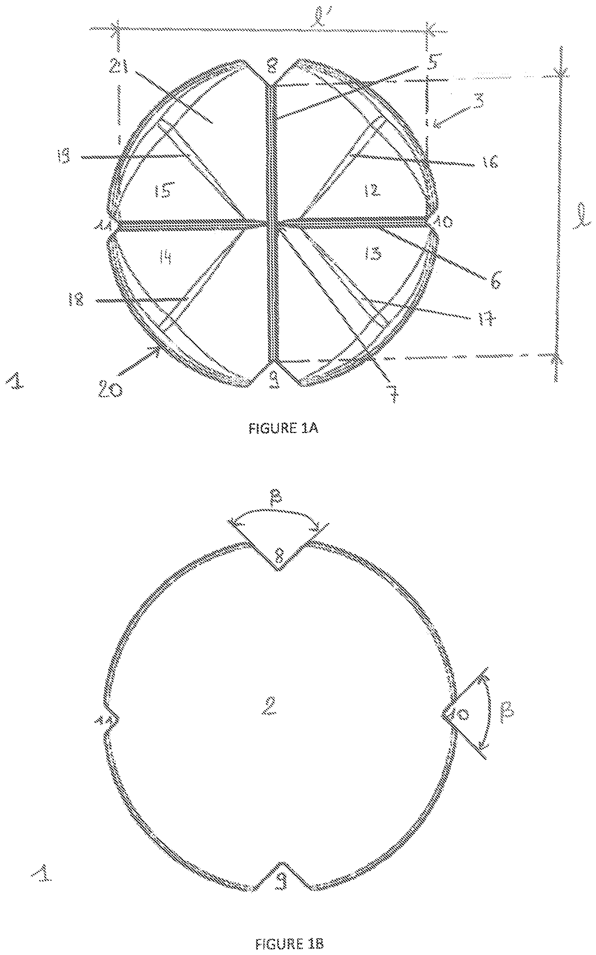

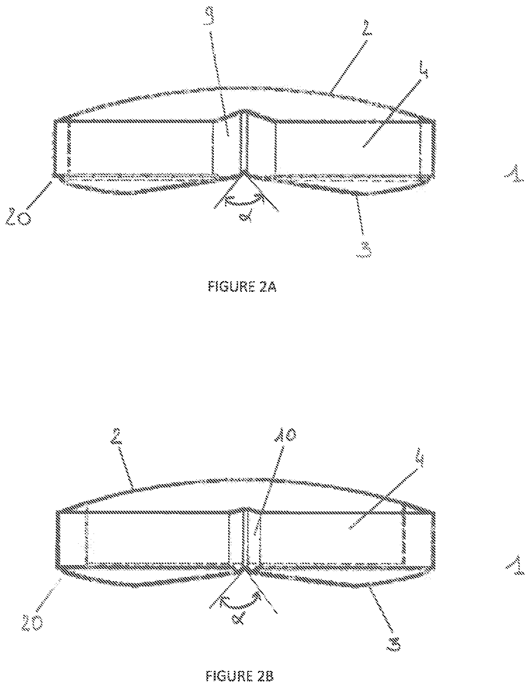

Dans l'exemple représenté aux figures 1 et 2, le comprimé présente une forme globalement circulaire. Bien entendu, d'autres formes peuvent être envisagées, et notamment une forme ovale, rectangulaire ou carré.

Le comprimé 1 comprend une face supérieure 2, une face inférieure 3 et une face latérale 4 s'étendant perpendiculairement entre les deux faces supérieure 2 et inférieure 3, sensiblement circulaires. La face supérieure 2 est convexe, comme cela est notamment visible sur les figures 2A et 2B, tandis que la face inférieure 3 est globalement concave.

Comme cela est visible sur la figure 1A, le comprimé 1 selon l'invention présente, sur sa face inférieure 3, deux gorges de sécabilité 5, 6, creusées dans l'épaisseur de ladite face inférieure 3. Par « épaisseur », on entend la dimension s'étendant perpendiculairement au plan d'observation de la face inférieure 3. Les gorges de sécabilité 5, 6 sont perpendiculaires l'une à l'autre. Une première gorge de sécabilité 5 s'étend de manière continue dans un diamètre de la face inférieure 3. La seconde gorge de sécabilité 6 est quant à elle discontinue. Plus précisément, la seconde gorge de sécabilité 6 s'interrompt au niveau de l'intersection 7 entre les deux gorges de sécabilité, située au centre de la face inférieure 3 du comprimé 1.

Les gorges de sécabilité 5, 6 ont une section en V, de manière à ce que la largeur desdites gorges de sécabilité soit décroissante depuis la surface externe de la face inférieure 3 vers l'intérieur du comprimé 1. Par « largeur », on entend la dimension des gorges s'étendant perpendiculairement à la plus grande dimension et dans le même plan. Bien entendu, il est possible de ménager des gorges de sécabilité présentant une section carré ou rectangulaire, par exemple, de manière à ce que la largeur desdites gorges soit constante. L'angle d'ouverture a des gorges de sécabilité 5, 6 est de 80°, +/- 5° (figures 2A et 2B).

Dans l'exemple représenté aux figures 1 et 2, les gorges de sécabilité 5, 6 ont des profondeurs identiques, mais il est possible de prévoir des profondeurs différentes entre les deux gorges. Dans ce cas, la gorge de sécabilité continue présente avantageusement la plus grande profondeur. Il est également possible de prévoir une profondeur variable le long d'une gorge de sécabilité, de manière par exemple à ménager une plus grande profondeur de gorge au centre du comprimé.

Les gorges de sécabilité 5, 6 ménagées sur la face inférieure 3 du comprimé 1 se prolongent sur la face latérale 4, de manière à y former des fentes de sécabilité 8, 9, 10, 11. Les fentes de sécabilité 8, 9, 10, 11 ont une section en V dont un angle d'ouverture β est d'environ 90°, +/- 5° (figure 1B).

Les deux gorges de sécabilité 5, 6 ont des longueurs 1 et différentes. Plus précisément, la gorge de sécabilité continue 5 a une longueur 1 inférieure à la longueur de la gorge de sécabilité discontinue 6. Pour obtenir une telle différence de longueur, alors que le comprimé présente une forme générale circulaire, les fentes de sécabilité 8, 9 s'étendant sur la face latérale 4 du comprimé 1 et dans le prolongement de l'une des gorges de sécabilité 5 sont plus profondes que les fentes de sécabilité 10, 11 s' étendant dans le prolongement de l'autre gorge de sécabilité 6.

Comme cela est visible sur la figure 1A, les gorges de sécabilité 5, 6 et les fentes de sécabilité 8, 9, 10, 11 permettent de diviser le comprimé 1 en quatre triangles 12, 13, 14, 15 identiques en termes de dimensions.

Dans l'exemple de réalisation décrit ici, la face inférieure 3 présente différentes pentes. Plus précisément, chaque triangle 12, 13, 14, 15 comprend une arête 16, 17, 18, 19 s'étendant depuis le bord externe 20 de la face inférieure 3 vers la gorge de sécabilité discontinue 6. Chaque triangle 12, 13, 14, 15 présente ainsi des pentes descendantes depuis l'arête 16, 17, 18, 19 correspondante vers les gorges de sécabilité. Avantageusement, les arêtes 16, 17, 18, 19 coupent la gorge de sécabilité discontinue 6 en amont de l'intersection 7 avec la gorge de sécabilité continue 5. La face inférieure 3 est ainsi partiellement concave. Plus précisément, la face inférieure 3 présente une zone de concavité 21 s'étendant sensiblement le long de la gorge de sécabilité continue 5, de sorte que l'épaisseur du comprimé 1 est la plus faible au niveau de l'intersection 7 entre les gorges de sécabilité 5, 6. La zone de concavité 21 est plus précisément délimitée par les arêtes 16, 17, 18, 19, depuis lesdites arêtes jusqu'au centre du comprimé 1.

Le rayon de courbure a de la zone de concavité est de 165°, +/- 5° (figure 2B).

Par ailleurs, le bord extérieur 20 de la face inférieure 3 du comprimé 1 est biseauté.

L'ensemble des dispositions présentées ci-dessus favorise la coupe du comprimé 1, de manière successive, en demis puis en quarts de comprimé. Il suffit pour cela de disposer le comprimé 1 sur une surface préférentiellement plane, face supérieure 2, convexe, vers le haut. Une pression exercée par un doigt sur la face supérieure 2 convexe du comprimé 1 permet de diviser le comprimé en deux parties identiques, la coupure se faisant le long de la gorge de sécabilité continue 5. Si cela s'avère nécessaire, il est ensuite possible d'exercer une pression avec le doigt sur la face supérieure d'un demi-comprimé, afin de couper ledit demi-comprimé en deux, le long de la gorge de sécabilité discontinue 6. On obtient ainsi deux quarts de comprimé identiques.

## Revendications

1. Comprimé sécable (1) comprenant une face supérieure (2) convexe, une face inférieure (3) au moins partiellement concave et une paroi latérale (4) s'étendant entre les faces supérieure et inférieure, deux gorges de sécabilité (5, 6) sont formées dans la face inférieure concave,
- lesdites gorges étant perpendiculaires l'une à l'autre et en continuité avec des fentes de sécabilité (8, 9, 10, 11) situées dans la paroi latérale,
- l'une des gorges de sécabilité est continue, s'étendant en une coupe unique entre les deux fentes de sécabilité, et l'autre gorge de sécabilité est discontinue, ladite gorge de sécabilité discontinue s'interrompant au niveau de l'intersection desdites gorges de sécabilité,
- chaque fente de sécabilité de la gorge de sécabilité continue présente une extension à l'intérieur du comprimé sécable plus profonde que l'extension de chaque fente de sécabilité de la gorge de sécabilité discontinue de sorte que la longueur de la gorge de sécabilité continue est plus petite que la longueur de la gorge de sécabilité discontinue pour favoriser la première casse du comprimé le long de la gorge de sécabilité continue.

2. Comprimé sécable selon la revendication 1, ledit comprimé ayant une forme circulaire cylindrique.

3. Comprimé sécable selon la revendication 1 ou 2, selon lequel les gorges de sécabilité de la face inférieure ont une section en V.

4. Comprimé sécable selon la revendication 3, selon lequel la section en V des gorges de sécabilité a un angle d'ouverture (β) compris entre 40° et 100°, plus préférentiellement égal à 80° +/-5°.

5. Comprimé sécable selon l'une des revendications précédentes, selon lequel les gorges de sécabilité de la face inférieure ont une profondeur comprise entre 1/5 et 3/10 de l'épaisseur du comprimé.

6. Comprimé sécable selon l'une des revendications précédentes, selon lequel le rayon de courbure (α) de la partie concave (21) de la face inférieure est compris entre 150° et 170°, préférentiellement égal à 165°, +/- 5°.

7. Comprimé sécable selon l'une des revendications précédentes, selon lequel les gorges de sécabilité divisent le comprimé en quatre triangles (12, 13, 14, 15) sensiblement identiques, la face inférieure de chacun des triangles présentant une arête (16, 17, 18, 19) s'étendant depuis le bord extérieur (20) vers une gorge de sécabilité, en amont de l'intersection (7) desdites gorges de sécabilité.

8. Comprimé sécable selon l'une des revendications précédentes, selon lequel un bord extérieur (250) de la face inférieure est biseauté.

9. Comprimé sécable selon l'une des revendications précédentes, selon lequel les fentes de sécabilité de la paroi latérales ont une section en V.

10. Comprimé sécable selon la revendication 9, selon lequel la section en V des fentes de sécabilité a un angle d'ouverture compris entre 70° et 120°, plus préférentiellement égal à 90° +/- 5°.

11. Comprimé sécable selon l'une des revendications précédentes, selon lequel ledit comprimé présente un plus grand diamètre compris entre 10 et 22 mm, préférentiellement entre 16 et 20 mm, plus préférentiellement de 18 mm.

## Patentansprüche

1. Teilbare Tablette (1), umfassend eine konvexe Oberseite (2), eine mindestens teilweise konkave Unterseite (3) und eine Seitenwand (4), die sich zwischen der Ober- und Unterseite erstreckt, wobei zwei Sollbruchrillen (5, 6) in der konkaven Unterseite ausgebildet sind,
- wobei die genannten Rillen senkrecht zueinander verlaufen und kontinuierlich in Sollbruchkerben (8, 9, 10, 11) übergehen, die in der Seitenwand angeordnet sind,
- eine der Sollbruchrillen ist durchgehend, wobei sie sich in einem einzigen Schnitt zwischen den beiden Sollbruchkerben erstreckt, und die andere Sollbruchrille ist unterbrochen, wobei die besagte unterbrochene Sollbruchrille am Schnittpunkt der besagten Sollbruchrillen unterbrochen ist,
- jede Sollbruchkerbe der durchgehenden Sollbruchrille weist im Inneren der teilbaren Tablette eine Erstreckung auf, die tiefer ist als die Erstreckung jeder Sollbruchkerbe der unterbrochenen Sollbruchrille, sodass die Länge der durchgehenden Sollbruchrille kleiner ist als die Länge der unterbrochenen Sollbruchrille, um das erste Brechen der Tablette entlang der durchgehenden Sollbruchrille zu begünstigen.

2. Teilbare Tablette nach Anspruch 1, wobei die Tablette eine kreisförmige zylindrische Form aufweist.

3. Teilbare Tablette nach Anspruch 1 oder 2, wobei die Sollbruchrillen der Unterseite einen V-förmigen Querschnitt aufweisen.

4. Teilbare Tablette nach Anspruch 3, wobei der V-förmige Querschnitt der Sollbruchrillen einen Öffnungswinkel (β) zwischen 40° und 100°, mehr bevorzugt von 80° +/-5°, aufweist.

5. Teilbare Tablette nach einem der vorstehenden Ansprüche, wobei die Sollbruchrillen der Unterseite eine Tiefe zwischen 1/5 und 3/10 der Dicke der Tablette aufweisen.

6. Teilbare Tablette nach einem der vorstehenden Ansprüche, wobei der Krümmungsradius (α) des konkaven Teils (21) der Unterseite zwischen 150° und 170° liegt, vorzugsweise gleich 165° +/- 5° beträgt.

7. Teilbare Tablette nach einem der vorstehenden Ansprüche, wobei die Sollbruchrillen die Tablette in vier im Wesentlichen identische Dreiecke (12, 13, 14, 15) unterteilen, wobei die Unterseite jedes der Dreiecke eine Kante (16, 17, 18, 19) aufweist, die sich vom Außenrand (20) zu einer Sollbruchrille erstreckt, stromaufwärtig des Schnittpunkts (7) der besagten Sollbruchrillen.

8. Teilbare Tablette nach einem der vorstehenden Ansprüche, wobei ein Außenrand (250) der Unterseite abgeschrägt ist.

9. Teilbare Tablette nach einem der vorstehenden Ansprüche, wobei die Sollbruchkerben der Seitenwand einen V-förmigen Querschnitt aufweisen.

10. Teilbare Tablette nach Anspruch 9, wobei der V-förmige Querschnitt der Sollbruchkerben einen Öffnungswinkel zwischen 70° und 120°, mehr bevorzugt von 90° +/- 5°, aufweist.

11. Teilbare Tablette nach einem der vorstehenden Ansprüche, wobei die Tablette einen maximalen Durchmesser zwischen 10 und 22 mm, vorzugsweise zwischen 16 und 20 mm, mehr bevorzugt von 18 mm aufweist.

## Claims

1. Scored tablet (1) comprising a convex top face (2), an at least partially concave bottom face (3) and a side wall (4) extending between the top and bottom faces, two score grooves (5, 6) being formed in the concave bottom face,
- said grooves being perpendicular to one another and in continuity with score slots (8, 9, 10, 11) located in the side wall,
- one of the score grooves is continuous, extending in a single cut between the two score slots, and the other score groove is discontinuous, said discontinuous score groove stopping at the intersection of said score grooves,
- each score slot of the continuous score groove has a deeper extension into the scored tablet than the extension of each score slot of the discontinuous score groove, so that the length of the continuous score groove is smaller than the length of the discontinuous score groove to promote first breakage of the tablet along the continuous score groove.

2. Scored tablet according to claim 1, said tablet having a circular cylindrical shape.

3. Scored tablet according to claim 1 or 2, wherein the score grooves on the bottom face are V-shaped in cross-section.

4. Scored tablet according to claim 3, wherein the V-shaped cross-section of the score grooves has an opening angle (β) of between 40° and 100°, more preferably equal to 80° +/-5°.

5. Scored tablet according to any of the preceding claims, wherein the score grooves on the bottom face have a depth of between 1/5 and 3/10 of the tablet thickness.

6. Scored tablet according to any of the preceding claims, wherein the radius of curvature (o) of the concave part (21) of the bottom face is between 150° and 170°, preferably equal to 165°, +/- 5°.

7. Scored tablet according to any of the preceding claims, wherein the score grooves divide the tablet into four substantially identical triangles (12, 13, 14, 15), the bottom face of each of the triangles having a ridge (16, 17, 18, 19) extending from the outer edge (20) toward a score groove, upstream of the intersection (7) of said score grooves.

8. Scored tablet according to any of the preceding claims, wherein an outer edge (250) of the bottom face is beveled.

9. Scored tablet according to any of the preceding claims, wherein the score slots in the side wall are V-shaped in cross-section.

10. Scored tablet according to claim 9, wherein the V-shaped cross-section of the score slots has an opening angle of between 70° and 120°, more preferably equal to 90° +/- 5°.

11. Scored tablet according to any of the preceding claims, wherein said tablet has a larger diameter of between 10 and 22 mm, preferably between 16 and 20 mm, more preferably 18 mm.
